Europäisches Patentamt

European Patent Office

Office européen des brevets

(19)

(11) Veröffentlichungsnummer: **0 224 813**
**B1**

(12) **EUROPÄISCHE PATENTSCHRIFT**

(45) Veröffentlichungstag der Patentschrift:
26.09.90

(51) Int. Cl.⁵: **C12Q 1/28**, G01N 33/72

(21) Anmeldenummer: 86116138.8

(22) Anmeldetag: 21.11.86

(54) Mittel zur Bestimmung von Peroxidase-Aktivität mit Stabilisator, Verfahren zur Herstellung und seine Verwendung.

(30) Priorität: 28.11.85 DE 3541979

(43) Veröffentlichungstag der Anmeldung:
10.06.87 Patentblatt 87/24

(45) Bekanntmachung des Hinweises auf die Patenterteilung:
26.09.90 Patentblatt 90/39

(84) Benannte Vertragsstaaten:
AT BE CH DE ES FR GB GR IT LI LU NL SE

(56) Entgegenhaltungen:
EP-A- 0 121 137
FR-A- 2 295 425
US-A- 4 077 772
US-A- 4 340 394
US-A- 4 504 579

(73) Patentinhaber: BEHRINGWERKE Aktiengesellschaft,
Postfach 1140, D-3550 Marburg 1(DE)

(72) Erfinder: Pauly, Hans Erwin, Dr., Finkenstrasse 1,
D-3563 Dautphetal(DE)
Erfinder: Schwarz, Herbert, Am Kirtenfeld 3,,
D-3557 Ebsdorfergrund(DE)

(74) Vertreter: Klein, Otto, Dr. et al, Hoechst AG Zentrale
Patentabteilung Postfach 80 03 20, D-6230 Frankfurt am
Main 80(DE)

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die vorliegende Erfindung betrifft ein Mittel zur Bestimmung von Peroxidase-Aktivität durch Farbreaktion sowie ein Verfahren zu seiner Herstellung und seine Verwendung.

Eine wesentliche Voraussetzung für die Einführung von Enzymimmunoassays, die hinsichtlich ihrer Nachweisempfindlichkeit radioimmunologischen Methoden ebenbürtig waren, stellte die Verfügbarkeit von stabilen Markerenzymen und entsprechenden hochempfindlichen Farbbildungsreagenzien dar, mit denen die katalytische Aktivität dieser Markerenzyme in meßtechnisch einfacher Weise zu registrieren war. Als besonders geeignete Markerenzyme erwiesen sich hierbei die Oxidoreductasen Glucoseoxidase und Peroxidase. Peroxidase-Reaktionen gehören generell zu den am häufigsten verwendeten enzymatischen Nachweisreaktionen. Alle z.B. in "Methods of Enzymatic Analysis", H.U. Bergmeyer, Ed., 3. Edition, Vol. 1, pp. 210-221, Verlag Chemie, Weinheim (1983) beschriebenen Verfahren beruhen, wie die Glucose-Bestimmung mittels Glucoseoxidase, auf der stöchiometrischen Erzeugung von Wasserstoffperoxid. Dieses kann dann in einer von Peroxidase katalysierten Oxidation eines farblosen Substrats zu einem farbigen Produkt umgesetzt werden, das einfach spektrophotometrisch quantifiziert werden kann.

Für diese von Peroxidase katalysierte Reaktion geeignete chromogene Systeme sind daher in großer Zahl untersucht und beschrieben (siehe Bergmeyer). Für die Bestimmung von Peroxidase-Aktivität in Enzymimmunoassays erfüllen nur wenige die Anforderungen, insbesondere hinsichtlich der Nachweisempfindlichkeit. Generell sollte ein chromogenes Substrat eine hohe Umsatzgeschwindigkeit erlauben und zu einem farbstabilen Produkt mit einem hohen molaren Extinktionskoeffizienten führen. Weiterhin sollten Substanzen bevorzugt werden, deren Handhabung kein gesundheitliches Risiko birgt. In kommerziellen Testkits für auf Peroxidase basierenden Enzymimmunoassays kommen vor allem o-Phenylendiamin (OPD) und 2,2'-Azinodi-(3-ethylenbenzothiazolinsulfonat-6) (ABTS) zum Einsatz. Sowohl OPD als auch ABTS sind wie die meisten Peroxidase-Substrate mutagen. Eine Gruppe der häufig verwendeten Substrate ist die des Benzidin-Typs, zu der auch Tetramethylbenzidin (TMB) gehört. TMB ist ein sicherer nichtmutagener Ersatz für carcinogene Peroxidase-Substrate des Benzidintyps wie Benzidin, Diaminobenzidin u.a. In zahlreichen Untersuchungen ergab sich kein Hinweis auf mutagene Eigenschaften dieses Benzidinderivats (Tetrahedron 30, 3299 (1976); Cancer Lett. 1, 39 (1975); J. Forensic Sci. 21, 816 (1976). TMB wurde seite 1974 von verschiedenen Anwendern zur Bestimmung der Pseudoperoxidase-Aktivität von Hämoglobin oder Cytochrome P 450 und bereits von Liem et al., Anal. Biochem. 98, 388-393 (1979) zur Detektion von Peroxidase-Aktivität in Immunkomplexen bei der Immunperoxidase-Färbetechnik verwendet. Diese Autoren weisen in ihrer Schlußfolgerung auf Seite 392 auf die guten Färbeeigenschaften, jedoch aber auch auf die geringe Löslichkeit des TMB in üblicherweise verwendeten Puffersystemen und auf die oxidative Zersetzung des TMB hin.

Ein Nachteil der bisher verwendeten Chromogensysteme unter Verwendung von TMB für den Nachweis von Peroxidase ist die geringe Stabilität des TMB im gebrauchs fertigen Ansatz. Auch in Abwesenheit von Peroxidase bewirkt Wasserstoffperoxid bereits in wenigen Stunden eine Farbbildung, die die Substratlösungen für eine Verwendung im Enzymimmunoassay aufgrund ihres hohen Blindwerts unbrauchbar werden läßt.

Es bestand daher der Bedarf, eine zur Bestimmung von Peroxidase geeignete, Tetraalkylbenzidin enthaltende Zubereitung zu finden, die den obenerwähnten Nachteil nicht hat. Eine solche Zubereitung, ihre Herstellung und ihre Verwendung ist Gegenstand der Erfindung.

Überraschenderweise wurde gefunden, daß eine hinsichtlich der Stabilität wesentlich verbesserte Zubereitung von Tetraalkylbenzidin erhalten werden kann, indem eine Lösung, die diese Verbindung oder eines ihrer Derivate enthält, mit Penicillin oder mit Penicillinderivaten, die durch saure Hydrolyse des Penicillins entstehen, in einer Konzentration zwischen 2.7 µmol/l und 2.7 mmol/l, vorzugsweise zwischen 13.5 µmol/l und 135 µmol/l versetzt wird und durch Mischen mit einem wässrigen Puffer, der eine geeignete Wasserstoffperoxid-Konzentration enthält, auf einen pH-Wert zwischen pH 2.5 und 6 eingestellt wird.

Ein Mittel zum Nachweis und zur Bestimmung von Peroxidase-Aktivität enthaltend ein Tetraalkylbenzidin oder eines seiner Salze, ein Peroxid oder ein Wasserstoffperoxid generierendes System, das dadurch gekennzeichnet ist, daß es eine oder mehrere Puffersubstanzen enthält, die einen pH-Wert im Bereich von 2.5 bis 3.9 einstellen, ist in der parallelen EP-A O 224 210 beschrieben.

Während Zubereitungen ohne den Zusatz von Penicillin oder -derivaten eine starke Erhöhung des Reagenzienblindwertes innerhalb von einer bis mehreren Stunden zeigen, wird durch einen solchen Zusatz die Haltbarkeit auf eine Dauer von einer bis mehreren Wochen erhöht.

Die Erfindung betrifft deshalb ein Mittel in Form einer flüssigen Zubereitung zum Nachweis und zur Bestimmung von Peroxidase enthaltend in einer wässrigen Lösung ein Tetraalkylbenzidin oder seine Salze, Penicillin oder seine durch schwach saure Hydrolyse aus dem bicyclischen Grundkörper des Penicillins entstehenden Abbauprodukte, beispielsweise Penicillinsäure oder 6-Aminopenicillansäure oder auch das nicht cyclische Penicillamin mit einem Gehalt von 2.7 µmol/l bis 2.7 mmol/l, Peroxide als Substrat für Peroxidase mit einem Gehalt von 0.5 bis 50 mmol/l und Puffersubstanzen.

Das Mittel kann durch Lösen einer festen Zubereitung hergestellt werden, beispielsweise eines Lyophilisats, eines Granulats oder einer Tablette, wobei die für die flüssige Zubereitung verwendeten Komponenten Tetraalkylbenzidin, Penicillin oder dessen Abbauprodukte, die Peroxide und die Puffersubstanzen in den Mengenverhältnissen enthalten sind, daß beim Auflösen in einem bestimmten Volumen vorwiegend wässrigen Lösungsmittels die Komponenten in den genannten Konzentrationen vorliegen. Die feste Zubereitung kann zusätzlich noch Zuschläge wie Gleitmittel, Füllstoffe und Sprengmittel, beispielsweise Polyäthylenglycol, Harnstoff und Bicarbonate enthalten.

Als Tetraalkylbenzidine sind vor allem solche verwendbar, die ein bis drei Kohlenstoffatome im Alkylteil enthalten, vorzugsweise 3,3',5,5'-Tetramethylbenzidin (TMB) oder sein Dihydrochlorid. Als Penicillin werden vorzugsweise Penicillin G und V verwendet. Als Peroxide sind Natriumperborat, Wasserstoffperoxid in flüssiger Form oder als festes Harnstoffaddukt oder auch ein Wasserstoffperoxid generierendes System bestehend aus D-Glucose und Glucoseoxidase geeignet, wobei eine Konzentration von 0.5 bis 10 mmol/l eingestellt wird. Als Puffersubstanzen werden lyotrope Substanzen wie Citrate und Acetate in Konzentrationen von 5 bis 100 mmol/l bevorzugt.

Bei der Herstellung einer Zubereitung in flüssiger Form wird Tetraalkylbenzidin in einer ersten sauren Lösung von verdünnter Salzsäure oder von Ameisensäure mit einem pH-Wert von 1.5 bis 2.0 gelöst. Vorzugsweise werden das Penicillin oder seine durch saure Hydrolyse entstehenden Abbauprodukte zu dieser Lösung zugesetzt.

Eine zweite, weniger saure Lösung wird hergestellt durch Lösen der Peroxide oder durch deren Eintragen im Falle der Verwendung einer Lösung von Wasserstoffperoxid. Zur Pufferung werden beispielsweise Essigsäure oder Mononatrium- beziehungsweise Monokalium-Citrat, deren pH-Wert mit Natriumhydroxid zwischen 3 und 6 reguliert werden kann, verwendet. Die genannten Abbauprodukte des Penicillins werden dieser zweiten Lösung zugesetzt, wenn diese oder ein Penicillin noch nicht der saureren Lösung zugesetzt worden waren.

Durch Vermischen der beiden Lösungen in einem bestimmten Verhältnis wird die gebrauchsfertige Zubereitung erhalten.

Die Erfindung wird durch die folgenden Beispiele näher erläutert.

Beispiele

1. Herstellung einer gebrauchsfertigen TMB-Substratzubereitung

Stammlösung 1: TMB-Dihydrochlorid wurde unter Rühren in einer Konzentration von 5 g/l, d.h. von 16 mmol/l in bidestilliertem Wasser gelöst und mit 5 normaler Salzsäure auf pH 1.5 eingestellt. Zu dieser Lösung wurde Penicillin G unter Rühren in einer Endkonzentration von 200 mg/l, d.h. von 0.56 mmol/l zugesetzt.

Zum Vergleich wurde Stammlösung 1 ohne Zusatz von Penicillin hergestellt.

Stammlösung 2: zu 900 ml bidestilliertem Wasser wurden 1.4 ml Eisessig, 1.5 ml 1 normale NaOH und 250 mg, d.h. 3 mmol $H_2O_2$ Harnstoff-Wasserstoffperoxid-Addukt zugesetzt. Nach vollständigem Lösen wurde mit bidestilliertem Wasser auf 1 l aufgefüllt.

Gebrauchslösung: Ein Volumenteil der Stammlösung 1 und 10 Volumenteile der Stammlösung 2 wurden miteinander vermischt.

Diese Lösung zeigte bei 650 nm eine optische Dichte von 0.025. Nach Zusatz des 5-fachen Volumens an 0.5 normaler Schwefelsäure betrug die bei 450 nm gemessene Extinktion 0.008.

2. Haltbarkeitsuntersuchung der Gebrauchslösung

Die TMB-Gebrauchslösung wurde gemäß Beispiel 1 hergestellt und im Kühlschrank bei 4-8° C gelagert. In gleicher Weise wurde eine gebrauchsfertige Lösung gemäß Beispiel 1, jedoch ohne Zusatz von Penicillin G, hergestellt und eingelagert. Nach definierter Lagerdauer wurden Aliquote dieser Gebrauchslösungen entnommen, mit dem 5-fachen Volumen an 0.5 normaler Schwefelsäure versetzt und die Extinktion bei 450 nm (TMB) registriert. Die Ergebnisse sind in der nachfolgenden Tabelle zusammengestellt.

Tabelle

Stabilität der Reagenzienblindwerte von Gebrauchslösungen zur Peroxidase-Bestimmung nach 6-facher Verdünnung mit 0.5 normaler Schwefelsäure
Reagenzienblindwerte nach Lagerung bei 4° C für

Reagenzienblindwerte nach Lagerung bei 4° C für

| | 0 h | 2 h | 24 h | 48 h | 7 d | 14 d |
|---|---|---|---|---|---|---|
| TMB-Ge-brauchs-lösung mit Penicillin | 0.008 | 0.015 | 0.006 | 0.008 | 0.014 | 0.024 |
| TMB-Ge-brauchs-lösung ohne Penicillin | 0.010 | 0.038 | 0.105 | 0.365 | – | – |

**Patentansprüche**

1. Mittel zum Nachweis und zur Bestimmung von Peroxidase-Aktivität enthaltend in einer wässrigen Lösung ein Tetraalkylbenzidin oder eines seiner Salze, ein Peroxid oder ein Wasserstoffperoxid generierendes System und eine Puffersubstanz, dadurch gekennzeichet, daß das Mittel Penicillin oder eines seiner durch saure Hydrolyse entstehenden Abbauprodukte enthält.

2. Mittel nach Anspruch 1, dadurch gekennzeichnet, daß das Tetraalkylbenzidin ein bis drei Kohlenstoffatome im Alkylteil enthält.

3. Mittel nach Anspruch 1, dadurch gekennzeichnet, daß das Tetraalkylbenzidin 3,3',5,5'-Tetramethylbenzidin ist.

4. Verfahren zur Herstellung eines Mittels nach Anspruch 1, dadurch gekennzeichnet, daß ein Tetraalkylbenzidin in einer Säure gelöst und ein Penicillin zugesetzt wird und diese Lösung mit einer weiteren Lösung, welche Peroxid und Puffersubstanzen enthält, vermischt wird.

5. Verfahren zur Herstellung eines Mittels nach Anspruch 1 zum Nachweis und zur Bestimmung von Peroxidase-Aktivität, dadurch gekennzeichnet, daß ein Tetraalkylbenzidin oder eines seiner Salze in einer wäßrigen Lösung von pH 1.5 bis 2 gelöst und dieser Lösung ein Penicillin oder ein durch saure Hydrolyse des Penicillins erhaltenes Abbauprodukt in einer Konzentration von 2.7 μmol/l bis 2.7 mmol/l, bezogen auf das Endvolumen, zugesetzt wird und diese Lösung mit einer weiteren wäßrigen Lösung, welche Peroxid und Puffersubstanzen enthält, vermischt wird.

6. Verfahren nach Anspruch 5, dadurch gekennzeichnet, daß im Falle der Verwendung von Abbauprodukten des Penicillins diese der Lösung zugesetzt werden, welche Peroxid und Puffersubstanzen enthält.

7. Verwendung des Mittels gemäß Anspruch 1 in analytischen Verfahren, in denen Peroxidase- oder Pseudoperoxidase-Aktivität nachgewiesen oder bestimmt wird.

**Claims**

1. An agent for the detection and for the determination of peroxidase activity, containing in an aqueous solution a tetraalkylbenzidine or one of its salts, a peroxide or a system generating hydrogen peroxide, and a buffer substance, which agent contains penicillin or one of its breakdown products produced by acid hydrolysis.

2. An agent as claimed in claim 1, in which the tetraalkylbenzidine contains one to three carbon atoms in the alkyl moiety.

3. An agent as claimed in claim 1, in which the tetraalkylbenzidine is 3,3',5,5'-tetramethylbenzidine.

4. A process for the preparation of an agent as claimed in claim 1, which comprises dissolving a tetraalkylbenzidine in an acid, and adding a penicillin, and mixing this solution with another solution which contains peroxide and buffer substances.

5. A process for the preparation of an agent as claimed in claim 1 for the detection and for the determination of peroxidase activity, which comprises dissolving a tetraalkylbenzidine or one of its salts in an aqueous solution of pH 1.5 to 2, and adding to this solution a penicillin or a breakdown product obtained by acid hydrolysis of the penicillin, in a concentration of 2.7 µmol/l to 2.7 mmol/l based on the final volume, and mixing this solution with another aqueous solution which contains peroxide and buffer substances.

6. The process as claimed in claim 5, wherein the breakdown products of the penicillin, where these are used, are added to the solution which contains peroxide and buffer substances.

7. The use of the agent as claimed in claim 1 in analytical methods in which peroxidase or pseudoperoxidase activity is detected or determined.

## Revendications

1. Moyen d'identification et de dosage de l'activité peroxydasique comprenant, en solution aqueuse, une tétraalkylbenzidine ou un de ses sels, un peroxyde ou un système générateur de peroxyde d'hydrogène, et une substance tampon, caractérisé en ce qu'il contient de la pénicilline ou un de ses produits de dégradation formés par hydrolyse acide.

2. Moyen selon la revendication 1, caractérisé en ce que la tétraalkylbenzidine contient de 1 à 3 atomes de carbone dans sa partie alkyle.

3. Moyen selon la revendication 1, caractérisé en ce que la tétraalkylbenzidine est la 3,3',5,5'-tétraméthylbenzidine.

4. Procédé de fabrication d'un moyen selon la revendication 1, caractérisé en ce que l'on dissout une tétraalkylbenzidine dans un acide, que l'on y ajoute une pénicilline et que l'on mélange cette solution avec une autre solution renfermant un peroxyde et des substances tampons.

5. Procédé de fabrication d'un moyen, selon la revendication 1, pour identifier et doser l'activité peroxydasique, caractérisé en ce que l'on dissout une tétraalkylbenzidine ou un de ses sels dans une solution aqueuse à pH 1,5 à 2, et que l'on ajoute à cette solution une pénicilline ou un produit de dégradation obtenu par hydrolyse acide de la pénicilline à une concentration de 2,7 µmole/l à 2,7 mmole/l, par rapport au volume final, puis que l'on mélange cette solution avec une autre solution aqueuse contenant du peroxyde et des substances tampons.

6. Procédé selon la revendication 5, caractérisé en ce que, dans le cas où l'on utilise des produits de dégradation de la pénicilline, on ajoute ces derniers à une solution contenant du peroxyde et des substances tampons.

7. Emploi du moyen selon la revendication 1 dans des procédés analytiques permettant d'identifier ou de doser l'activité de la peroxydase ou de la pseudoperoxydase.